# EUROPEAN PATENT APPLICATION

(11) **EP 3 263 181 A1**
(43) Date of publication of application: **03.01.2018**
(21) Application number: 17178992.8
(22) Date of filing: 30.06.2017
(51) Int. Cl.: A61Q 17/00, A61K 8/43, A61K 8/42, A61Q 19/10, A61K 6/00, A61K 31/155, A61K 31/167, C11D 3/48, C11D 1/10, C11D 1/88, C11D 1/75

(54) **STABLE AND SAFE ANTISEPTIC SOLUTION COMPRISING CHLORHEXIDINE**

(30) Priority: 30.06.2016 US 201662356975 P
(71) Applicant: AVA, Inc., Willowbrook, IL 60527 (US)
(72) Inventor: Chakroborty, Arjun, Willowbrook, IL Illinois 60527 (US)
(74) Representative: FRKelly

(57) **Abstract**

A stable and safe skin antiseptic solution comprises chlorhexidine, pharmaceutically acceptable salts of chlorhexidine (e.g. chlorhexidine digluconate), or a mixture thereof, dimethyl lauramide/myristamide, and water. Said solution is essentially free of coconut oil diethanolamine concentrate ("cocamide DEA"), a known carcinogen. The active antiseptic ingredient, chlorhexidine (or its salts), is a di(4-chlorophenyldiguanido) compound. The functions of dimethyl lauramide/myristamide in said solution include, but not limited to, an aesthetics enhancer, a foam stabilizer, a solubilizer, and a fragrance. Said solution is specifically formulated to be safe with a reduced risk of carcinogenicity, and be capable of producing a stable and long lasting foam. The active antiseptic agent is evenly distributed in said solution and in said foam, respectively.

## Description

### FIELD OF THE INVENTION

The present invention relates to an antimicrobial composition, such as chlorhexidine digluconate solution, that is specifically formulated to be safe for human use with a reduced risk of carcinogenicity, be capable of producing stable, long lasting foam in foaming applications, and be capable of providing an even distribution of the active antiseptic agent. The composition has been found to (1) increase overall stability of a variety of chlorhexidine antiseptic products, and (2) decrease impurities over the lifetime of chlorhexidine products. The composition can be used in the healthcare profession as a hand preparation or as a pre-surgical scrub without requiring a secondary application. The present invention is also useful in consumer markets as an antimicrobial solution for general cleansing.

### BACKGROUND

Hand and/or body washing is an essential component of infection control activities. Consumers typically wash their hands on a regular basis to control the spread of potentially infectious agents: including but not limited to bacteria, fungi, and viruses. Healthcare professionals attending to patient care are subject to higher standard of hand disinfection and must wash their hands to control the spread of infectious agents from patient to patient. In particular, before any surgical procedures, healthcare professionals have to perform surgical hand scrubbing and patient preoperative skin preparations.

Hand and/or body washing procedures are performed in several ways. Commonly used antimicrobial compositions include, but not limited to, antimicrobial bar soaps, antimicrobial liquid or foaming liquid soap, skin disinfecting alcohol-based agents, and the like. Chlorhexidine digluconate ("chlorhexidine gluconate" or "CHG")-based solutions for hand and/or body washing are typically used in the human and animal healthcare industries in the United States of America, though recently there has been minor proliferation into the general consumer market(s).

A large portion of the commercially available hand and/or body washing solutions with chlorhexidine digluconate as the active disinfecting agent also contain diethanolamide, such as coconut oil diethanolamine concentrate ("cocamide DEA") as an excipient to increase viscosity of the solution, or as a foam stabilizer, and/or for other purposes. The specific function of such an excipient depends on individual compositions. A significant drawback is that cocamide DEA is a known carcinogen.

In addition to the concerns of carcinogenicity of diethanolamide excipients, it is difficult to formulate a CHG based solution with diethanolamide excipients that will feel, lather, and foam similar to ordinary soaps and detergents currently and previously available on the market. Therefore, it is desirable to produce a disinfecting (i.e. antiseptic/ antimicrobial) skin composition containing a chlorhexidine compound such as CHG with a new excipient as a replacement of diethanolamide excipients. Ideally, the replacement excipient should not only be safe to use, but also give the disinfecting composition a desirable feel and better lathering/foaming characteristics.

### SUMMARY

The present invention, according to the embodiments disclosed herein, provides antimicrobial effectiveness. The present invention is specifically formulated to be safe with a reduced risk of carcinogenicity, and is capable of producing a stable and long lasting foam (in foaming applications). In the present invention, the active antiseptic agent is generally evenly distributed.

In one embodiment, a skin disinfecting composition comprises, at least, an antiseptic agent such as chlorhexidine digluconate, a thickening and/or foaming agent such as dimethyl lauramide/myristamide ("DLM"), and water. DLM (as NINOL®CAA) is a dimethyl amide mixture containing a carbon chain of C₁₂ or C₁₄ commercially available from Stepan Company (Northfield, Illinois, USA). Solubility of dimethyl lauramide/myristamide in chlorhexidine digluconate solutions is best achieved with a 1:1 solution of water with an alcohol, aldehyde, or ketone. The disinfecting composition is used in a method of disinfecting a substrate or surface, such as a hand (i.e. skin surface), comprising the step of applying an effective amount of the disinfecting composition to the skin surface.

Preferably, said skin disinfecting composition may further comprise a colorant or a fragrance. Specifically, said skin disinfecting composition comprises: (a) up to about 21% by weight of an antiseptic agent, such as CHG; (b) from about 0% to about 10% by weight of a thickening and/or foaming agent; (c) from about 0.00005% to about 5% by weight of a fragrance; (d) optionally from about 0.00005% to about 1% by weight of a colorant; and (e) from about 5% to about 95% by weight of water. Said skin disinfecting composition is useful in providing substantial antimicrobial effectiveness meeting the expectations of contemporary users.

Another attribute of said skin disinfecting composition is its ability to maintain a dense and stable foam, when utilized in foaming applications such as skin washing or surface cleansing. Another advantage of said skin disinfecting composition is its compatibility with chlorohexidine digluconate, which can provide further potential reduction of microbial flora on the substrate, such as skin, rendering the composition useful in healthcare markets. A further advantage of said skin disinfecting composition is the potential to provide long-term residual antimicrobial activity and maintain such activity on the applicant's skin to prevent the bacteria from growing back to the baseline of normal skin flora population. Further, said skin disinfecting composition can also be used as a general purpose hand wash to decontaminate the hands of healthcare professionals before examining any patient. Said skin disinfecting composition can be used by general consumers.

Healthcare professionals perform a routine hand scrubbing procedure multiple times a day. Consumers also typically routinely wash their hands multiple times a day. Commonly used antiseptic solutions for such purposes contain chlorhexidine digluconate as an antiseptic agent. Chlorhexidine digluconate solutions disinfect the skin and bind to the skin, and thus provide persistent activity. Since healthcare professionals and consumers alike scrub or wash their hands many times a day, the chemical compounds may build up on their skin and accumulate throughout the day. Said skin disinfecting composition includes a thickening agent and/or foaming agent that may be used to distribute the disinfecting compound evenly, allowing for a uniform build-up of the disinfecting agent on the application area. Said skin disinfecting composition would be ideal for both routine uses throughout the day by healthcare professionals and less frequent uses by general consumers.

Said antimicrobial compositions are typically packaged in a container. Typically, pressure is applied to a container by manually compressing the container until product is dispensed. A pump, for example electric-powered, hand-powered, foot-powered, or by other power means, may be used to create an increased pressure within the container to dispense the product. The positive pressure difference across the container wall can force said skin disinfecting solution out of the container. Alternatively, said skin disinfecting may be drawn out of the container through a straw or similar devices in conjunction with pump means. To achieve such desirable properties, said skin disinfecting composition has to satisfy certain physical characteristics. Some examples of these properties include: viscosity in the range of ≤ 2500 cps (typically <100 cps), a thickening and/or foaming agent in a concentration range of 0 - 10% w/w (typically 0-2.5%), and an antiseptic agent as an active ingredient in a concentration range of 0 - 20% w/w (typically 0-5%). In a preferred embodiment, the skin disinfecting composition contains dimethyl lauramide/myristamide as a thickening and/or foaming agent. Said skin disinfecting composition is effective against microorganisms resulting in persistent bacterial reduction and is skin-tolerable for regular, consistent, and repeated topical administrations. When applied in foaming application, said skin disinfecting composition is, among other desirable properties, relatively non-irritating, dispensable, and latherable and has stable foaming capabilities, especially when applied to human skin surfaces.

### BRIEF DESCRIPTION OF THE DRAWINGS

FIG. 1 illustrates, in one embodiment, viscosities of a series of aqueous test solutions having different viscosity enhancers: dimethyl lauramide/myristamide, cocamide MIPA, cocamide MEA, cocamide DEA, cocamide DIPA, and cocamidopropryl betaine.
FIG. 2 illustrates, in one embodiment, foaming properties of a series of aqueous test solutions having different viscosity enhancers: dimethyl lauramide/myristamide, cocamide MIPA, cocamide MEA, cocamide DEA, cocamide DIPA, and cocamidopropryl betaine.
FIG. 3 illustrates, in one embodiment, foam stability (foam height in mm of foam) of chlorhexidine digluconate (CHG) antiseptic solutions containing dimethyl lauramide/myristamide or cocamide DEA. The replacement ratios between dimethyl lauramide/myristamide and cocamide DEA are 1:1 and 1:2.
FIG. 4 illustrates a stability table for a foaming composition of Example 3 containing about 0.75% CHG by weight, including data columns: Time point; Visual Inspection; Physical appearance; pH; Specific gravity (g/cc); Viscosity cps @ 25°C (Spindle 1, or S61@60 rpm); Foam 'height' (i.e. vol. in cc); % IPA by weight; % CHG by weight; PCA (p-chloroaniline) in ppm; % Total organic impurities; and Microbiological assay (<=10 CFU/mL).

### DETAILED DESCRIPTION

The present invention provides an antiseptic solution composition, which comprises, at least, an active antiseptic agent such as chlorhexidine digluconate, a thickening and/or foaming agent such as dimethyl lauramide/myristamide (DLM), and a solvent such as water. In addition, said composition may further contain one or more of emulsifiers, preservatives, moisturizers, emollients, ethyl alcohol, isopropyl alcohol, colorants, fragrances, and other acceptable excipients.

The term "antiseptic agent" as used herein refers to a chemical substance that can be applied to humans or animals that retards or halts the growth of microorganisms without necessarily destroying them. Several useful antiseptic agents include, but are not limited to, alcohols such as ethanol and isopropanol, boric acid or other borates, menthol, hydrogen peroxide, iodides, phenol, and the like. Also contemplated is chlorhexidine gluconate (CHG), also named as 1,6-bis(4-chloro-phenylbiguanido)hexane, as generally commercially available, and similar molecules such as octenidine dihydrochloride (IUPAC name *N*-octyl-1-[10-(4-octyliminopyridin-1-yl)decyl]pyridin-4-imine dihydrochloride).

The term "thickening and/or foaming agent" as used herein refers to chemical substances used to prepare a stable liquid formulation, including a gas in liquid. Useful foaming agents can include dimethyl lauramide/myristamide (DLM) and the like.

Emulsifiers, preservatives, moisturizers, emollients are also contemplated as useful components as known in the art.

The term "essentially free" or "substantially free" as used herein refers to materials sold by the manufacturer and labeled as "free of" a residue or contaminant. It is understood that standard analytical testing will be employed as understood by an analytical chemist measuring performance chemicals or a qualified formulations chemist. In an embodiment, essentially free (or substantially free) can mean at least greater than about 95 % purity (with respect to a measurable impurity), or greater than 96%, 97%, 98%, 99%, or 99+% by weight.

In one embodiment, the present invention relates to a skin disinfecting composition that provides antimicrobial effectiveness and is specifically formulated to have a reduced risk of carcinogenicity. Coconut oil diethanolamine concentrate has been extensively used as a viscosity, texture, and foam enhancer in topically administered compositions. Research, however, indicates that the biologic effects of diethanolamine appear to associate with its incorporation into phospholipids in place of ethanolamine. Without intending to be bound by theory, it is thought that the pathways of phospholipid biosynthesis using ethanolamine and choline are highly conserved and essentially the same in all mammals, as is the function of phospholipids as structural components of cell membranes and their role as second messengers. The incorporation of diethanolamine into phospholipids in suitably exposed mammalian species is indicative of potential inherent carcinogenic properties.

The United States Environmental Protection Agency published a report entitled Cancer Assessment Document, Evaluation of the Carcinogenic Potential of Cocamide Diethanolamine (DEA). (NTP, 2001; U.S. EPA, January 2001). The report concludes that the cocamide DEA causes cancer. The report concludes that cocamide diethanolamine is "'likely to be carcinogenic to humans' based on the occurrence of liver and kidney tumors in male and liver tumors in female B6C3F1 mice." Specifically, the U.S. EPA described a study of male mice treated with cocamide diethanolamine, which shows significant increases in the incidences of combined renal tubule carcinomas and adenomas, hepatoblastomas, and combined hepatocellular adenomas, carcinomas, and hepatoblastomas. The U.S. EPA also described a study of female mice treated with cocamide diethanolamine, which shows significant increases in the incidences of hepatocellular carcinomas and combined hepatocellular adenomas, carcinomas, and hepatoblastomas. For multiple reasons, including those described in the U.S. EPA report, the State of California Office of Environmental Health Hazard Assessment added both diethanolamine and coconut oil diethanolamine (concentrate) to the State of California Proposition 65 list, mandating that all commercial uses of diethanolamine and coconut oil diethanolamine be labeled to be "known to the State of California to cause cancer."

Said antiseptic solution composition contains DLM. DLM can be used, for example, as an aesthetic enhancer, a fragrance solubilizer, and/or a viscosity enhancer. DLM is essentially free of or with untraceable amounts of diethanolamine, essentially free of or with untraceable amounts of ethylene oxide, and cold-mixable with water. As opposed to diethanolamide excipients (e.g. cocamide DEA), DLM presents several desirable properties. DLM (commercially available from Stepan Company, Northfield, IL, USA) is essentially free of both diethanolamine and ethylene-oxide. Diethanolamine and ethylene-oxide in DLM are only traceable on a PPM scale. To date, no substantial concerns have arisen regarding potential carcinogenicity of DLM. In addition to apparent limited carcinogenicity, DLM provides better solubilizing properties and foam building properties and is chemically inert in a solution containing chlorhexidine digluconate (chemically compatible with CHG).

Said skin disinfecting composition desirably comprises at least an antiseptic or similar agent such as chlorhexidine digluconate, a thickening and/or foaming agent such as dimethyl lauramide/mrystamide, and water. The disinfecting composition is used in a method of disinfecting a substrate or surface, such as hand (skin surface), comprising the step of applying an effective amount of the disinfecting composition to the skin surface.

Said skin disinfecting composition may further comprise a colorant or a fragrance. Preferably, said skin disinfecting composition comprises by weight: (a) up to 21% by weight of chlorhexidine digluconate based on the total weight of the composition; (b) up to 10% by weight of a thickening and/or foaming agent based on the total weight of the composition; (c) up to 5% by weight of a fragrance based on the total weight of the composition; (d) optionally up to 1% by weight of a colorant based on the total weight of the composition; and (e) from about 5% to about 95% by weight of water based on the total weight of the composition. Said skin disinfecting composition is useful in providing substantial antimicrobial effectiveness whilst effectively satisfying the expectations of the contemporary users of antimicrobial solutions.

Another attribute of said skin disinfecting composition is its ability to maintain a dense and stable foam, when utilized in foaming applications. Another advantage of said skin disinfecting composition is its compatibility with chlorohexidine digluconate allowing for further potential reduction of microbial flora on the skin. A further advantage of the skin disinfecting composition of the present invention is the potential to provide long-term residual antimicrobial activity on the applicant's skin to prevent bacteria growing back to the base line of normal skin flora population. Further, the skin disinfecting composition of the present invention can be used both by healthcare professionals as a general purpose hand wash to decontaminate the hands before examining any patients and by general consumers.

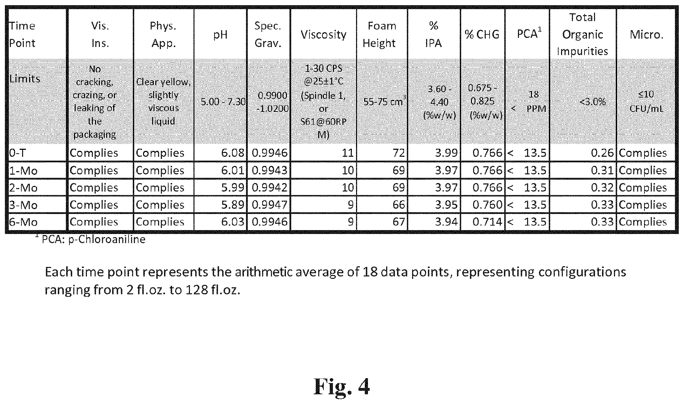

Said disinfecting solution has been placed under accelerated stability study conditions (40 °C and 75±5% relative humidity) for six (6) months. The test results demonstrated that said disinfecting solution is stable and consistent under the stressed conditions. Said disinfecting solution maintained its integrity after several (≥5) freeze-thaw cycles and also passed light degradation tests. See, Figure 4.

### Materials:

Cocamide DEA is a coconut oil diethanolamine concentrate produced via the 1:1 reaction. Cocamide DEA can be used as a viscosity booster and foam booster and is widely applied in liquid hand soap products. Cocamide DEA is a liquid at room temperature having a clear to yellowish color. 1% cocamide DEA aqueous solution has a pH of 9.3. At 25 °C, cocamide DEA solution has a viscosity of 700 CPS. The average molecular mass of cocamide DEA is about 280 g/mol. Dimethyl Lauramide / Myristamide (DLM) is a dimethyl amide containing saturated, vegetable-derived C₁₂ or C₁₄ carbon chains. DLM is a clear to pale yellowish liquid at room temperature. 5% DLM in 1:1 isopropyl alcohol/water mixture has a pH of 4.6. At 25 °C, DLM solution has a viscosity of 8 CPS. The average molecular mass of DLM is about 234 g/mol.

The skin aseptic solutions described herein may be further understood in connection with the following Examples.

### Example 1 - Performance Test No. 1

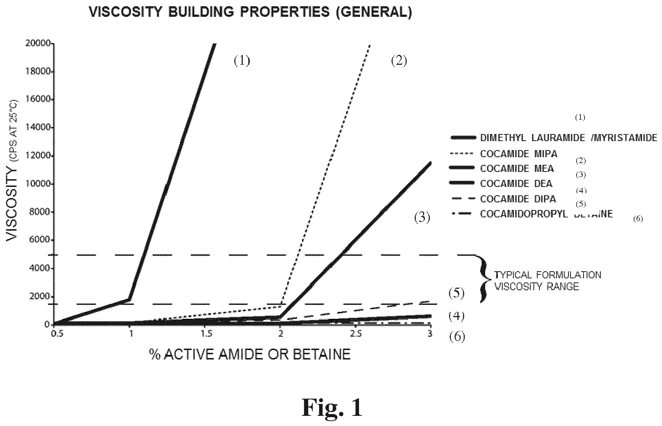

The viscosity enhancing performance of DLM was examined in a test solution, which comprises 12% active sodium laureth sulfate (2 moles), 1.5% sodium chloride, and amide or betaine in the amount indicated in Figure 1. The tested amide/betaine concentrations were 0.05%, 1%, 1.5%, 2%, 2.5%, 3.0%, 3.1% 3.25%, and 3.5% by weight, respectively. The testing results suggest that DLM is a superior viscosity enhancer, allowing the solution to reach typical composition viscosity range with a smaller molar amount of DLM than several common viscosity enhancers, e.g. cocamide DEA. As shown in Fig. 1, in a series of test solutions containing different viscosity enhancers, the amount of DLM required to achieve a similar viscosity in an equivalent chlorhexidine digluconate solution is only about 50% of the amount of cocamide DEA. The preferred concentrations of CHG in certain commercial products can include 0.75%, 2%, and 4% by weight, respectively, as shown in Example 4.

Viscosity was tested as per United States Pharmacopeia <911> and/or <912>, or comparable. To date, product was tested as per an accelerated lifecycle at 40 °C and 75±5% relative humidity, with sampling taken at Time of Manufacture (0-time), 1-month from manufacture, 2-months from manufacture, 3-months from manufacture, and 6-months from manufacture.

### Example 2 - Performance Test No. 2

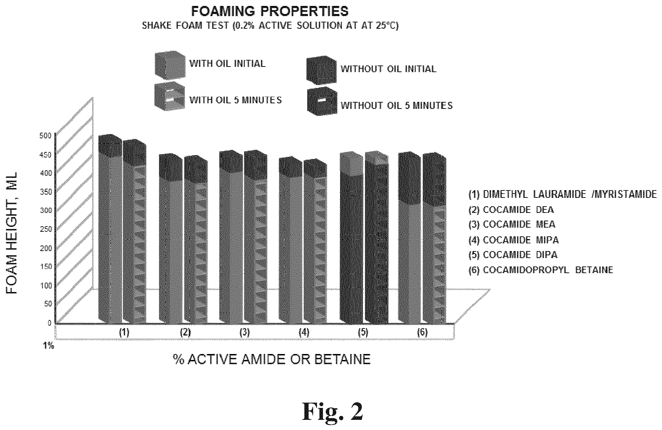

The foaming enhancing properties and stabilizing performance of DLM were examined in a test solution having 12% active sodium laureth sulfate (2 moles), 1.5% sodium chloride, and amide or betaine in the amount indicated in Figure 2. The tested amide/betaine concentrations were 0.05%, 1%, 1.5%, 2%, 2.5%, 3.0%, 3.1% 3.25%, and 3.5% by weight, respectively. The test results suggest that dimethyl lauramide/myristamide is a superior foam stabilizer, allowing the solution to reach up to about 10% greater foam heights. Additional advantages of using DLM include a clearer solution and a milder feel to many users due to a smaller amount of this excipient required in the solution.

ASTM D1173 test was performed, or comparable. To date, product was tested as per an accelerated lifecycle at 40 °C and 75±5% relative humidity, with sampling taken at Time of Manufacture (0-time), 1-month from manufacture, 2-months from manufacture, 3-months from manufacture, and 6-months from manufacture.

### Example 3 - Performance Test No. 3

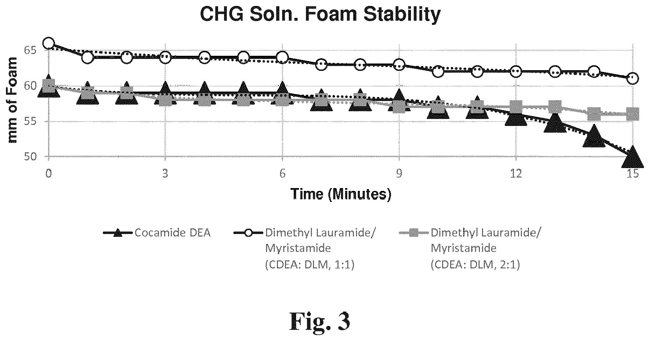

The foaming enhancing properties and stabilizing properties of DLM as a replacement for cocamide DEA were further examined in a 0.75% by weight CHG antiseptic solution utilizing foaming shake test. As shown in Fig. 3, the test results obtained from 1:1 / DLM:cocamide DEA replacement experiment suggest that DLM is a superior foam stabilizer, allowing the solution to reach up to about 10% greater foam heights whilst being more persistent. In a 1:2 / DLM:cocamide DEA replacement experiment, both chlorhexidine antiseptic solutions generated similar foam heights for up to 12 minutes. After 12 minutes, the chlorhexidine antiseptic solution containing DLM could still largely maintain the foam height, whereas the foam height in the solution containing cocamide DEA dropped rapidly. Additional benefit of using DLM includes a clearer solution and a potentially milder feel due to a smaller amount of this excipient required in the solution.

In one embodiment, the foaming solution may contain from about 0.5% to about 5% by weight CHG.

In another embodiment, the foaming solution may contain from about 0.75% to about 0.85% by weight CHG. In a preferred embodiment, the foaming solution may contain from about 0.75% to about 0.80% by weight CHG. In a particularly preferred embodiment, the foaming solution may contain about 0.75% by weight CHG. See Table 1.

**Table 1. CHG Foamer Solution**

| **Material** | **Material CAS #¹** | **Composition (% w/w)** |
|---|---|---|
| Water | 7732-18-5 | 84.6844 |
| Hydroxyethylcellulose | 9004-62-0 | 0.3000 |
| Lauramine Oxide | 1643-20-5 | 3.5000 |
| Dimethyl Lauramide /Myristamide (DLM) | 3007-53-2 3015-65-4 | 1.2500 |
| Ricinolamidopropyl Trimethyl Ammonium Chloride *("Surfactol Q1")* | 127311-98-2 57-55-6 | 2.0000 |
| Citric Acid, anhydrous | 77-92-9 | 0.0600 |
| Chlorhexidine Digluconate Soln. (20% w/w) (a.k.a. Chlorhexidine gluconate Soln.) | 18472-51-0 | 4.0900 |
| Isopropyl Alcohol, 99% | 67-63-0 | 4.0400 |
| Fragrance | *Not Available* | 0.0700 |
| Tartrazine *("FD&C Yellow 5")* | 1934-21-0 | 0.0055 |
| Scarlet GN *("FD&C Red 4")* | 4548-53-2 | 0.0001 |
| Total: | | 100.0000 |

| | | |
|---|---|---|
| ¹ CAS#s provided may not be exhaustive | | |

Foam testing: 5 mL of product was inserted into a 100 mL graduated cylinder with an inner diameter of 30.0±1.0 mm. The cylinder was covered and agitated vigorously for 60 seconds. Foam height was measured over a set period of time, dependent upon the product tested. Repeat as necessary. See, Figure 4.

For the above composition foam may be produced having a density of about 0.1 g/cc to about 1.5 g/cc and/or average bubble number distribution of about 1.0x10⁵/cc to about 1.0x10⁵/cc producing stable foaming. In another embodiment foam is produced having a density of about 0.25±0.05 g/cc and/or average bubble number distribution of 5.0x10⁶±1.0x10⁶/cc producing stable foaming over at least 15 minutes, or longer.

### Example 3A - Performance Test No. 4

The foaming enhancing properties and stabilizing properties of DLM as a replacement for cocamide DEA have been further examined in a 2% by weight CHG antiseptic solution utilizing foaming shake test. The test results obtained from 1:1 / DLM:cocamide DEA replacement experiment suggest that DLM is a superior foam stabilizer, allowing the solution to reach up to greater foam heights whilst being more persistent.

### Example 3B - Performance Test No. 5

The foaming enhancing properties and stabilizing properties of DLM as a replacement for cocamide DEA have been further examined in a 4% by weight CHG antiseptic solution utilizing foaming shake test. The test results obtained from 1:1 / DLM:cocamide DEA replacement experiment suggest that DLM is a superior foam stabilizer, allowing the solution to reach up to greater foam heights whilst being more persistent.

### EXAMPLE 4

### Purpose

This experiment was designed to test the viability of replacing cocamide DEA currently used in chlorhexidine digluconate solutions with dimethyl lauramide/myristamide.

### Production of testing batches

The testing batches were of the 760±5 kg size. This equates to an approximate batch volume of 200 US Gallons. Proposed manufacturing batch sizes ranging from 760 kg to 7600 kg are presented below, in Table 2.

**Table 2. Testing Batch Sizes Composition**

| **Batch Size in Kilograms** | **Batch Size in US Gallons** | **Batch Size in Liters** |
|---|---|---|
| ∼760 | 200 | ∼757 |
| ∼7600 | 2000 | ∼7570 |

The results of concentration laddering experiments indicate that the performance of DLM is superior to cocamide DEA and the amount of DLM required to achieve similar viscosity and foaming height in a chlorhexidine digluconate solution is approximately 50% by weight of cocamide DEA. Since the required amount of DLM is smaller than that of cocamide DEA in a chlorhexidine digluconate solution, additional purified water was added to the solution to compensate the difference in mass. No additional changes were made to the qualitative or quantitative composition of the skin aseptic solution. A comparison of the qualitative and quantitative compositions describes below.

In order to demonstrate the compatibility of DLM with chlorhexidine digluconate, a pilot batch of 2% chlorhexidine digluconate solutions (with or without 4% by weight Cocamide DEA) were prepared and tested according to currently approved finished product specification criteria. The test results of the 2% CHG solutions are presented in Table 3.

**Table 3. Test Results of 2% CHG Solutions.**

| **Test** | **Specifications** | **Results** | | **Conformation to Specifications** |
|---|---|---|---|---|
| | | **Cocamide DEA** | **Dimethyl Lauramide / Myristamide** | |
| Physical Appearance | Clear to very slight haze, amber liquid with no particulate | Conforms | Conforms | Conforms |
| pH | 6.00-7.50 | 6.52 | 6.46 | Conforms |
| Specific Gravity | 0.9900 - 1.0300 | 1.0038 | 0.9981 | Conforms |
| Viscosity¹ | 3-30 | 17.0 | 12.5 | Conforms |
| CHG² | 1.95-2.20 | 2.13 | 2.10 | Conforms |
| IPA² | 4.00-4.80 | 4.60 | 4.63 | Conforms |
| p-Chloroaniline³ | ≤ 50 | < 13.5 | < 13.5 | Conforms |

| | | | | |
|---|---|---|---|---|
| ¹ Units: CPS@ 25±1°C (Utilize Spindle 1 [S61] @ 60 RPM) ² Units: % w/w ³ Units: PPM | | | | |

In order to demonstrate the compatibility of DLM with chlorhexidine digluconate, a pilot batch of 4% chlorhexidine digluconate solutions (with or without 4% by weight Cocamide DEA) were prepared and tested according to currently approved finished product specification criteria. The test results of the 4% CHG solutions are presented in Table 4.

**Table 4. Test Results of 4% CHG Solutions**

| **Test** | **Specifications** | **Results** | | **Conformation to Specifications** |
|---|---|---|---|---|
| | | **Cocamide DEA** | **Dimethyl Lauramide / Myristamide** | |
| Physical Appearance | Clear to very slight haze, amber liquid with no particulate | Conforms | Conforms | Conforms |
| pH | 6.00-7.50 | 6.70 | 6.29 | Conforms |
| Specific Gravity | 0.9900 - 1.0300 | 1.0106 | 1.0050 | Conforms |
| Viscosity¹ | 3-30 | 11.0 | 8.7 | Conforms |
| CHG² | 1.95-2.20 | 4.14 | 4.25 | Conforms |
| IPA² | 4.00-4.80 | 4.55 | 4.53 | Conforms |
| p-Chloroaniline³ | ≤ 50 | < 13.5 | < 13.5 | Conforms |

| | | | | |
|---|---|---|---|---|
| ¹ Units: CPS@ 25±1°C (Utilize Spindle 1 [S61] @ 60 RPM) ² Units: % w/w ³ Units: PPM | | | | |

In order to demonstrate the compatibility of DLM with chlorhexidine digluconate, a pilot batch of 2% chlorhexidine digluconate liquid solutions (with or without 4% by weight Cocamide DEA) were prepared and tested according to currently approved finished product specification criteria. The test results of the 2% CHG liquid solutions are presented in Table 5. Compared to the above solutions, CHG liquid solutions may contain, among other changes, 0.6% by weight hydroxyethylcellulose, which acts as a thickening agent CHG liquid solutions are more viscous than corresponding solutions above.

**Table 5. Test Results of 2% CHG Liquid Solutions.**

| **Test** | **Specifications** | **Results** | | **Conformation to Specifications** |
|---|---|---|---|---|
| | | **Cocamide DEA** | **Dimethyl Lauramide / Myristamide** | |
| Physical Appearance | Clear to very slight haze, amber liquid with no particulate | Conforms | Conforms | Conforms |
| pH | 6.00-7.50 | 6.45 | 6.35 | Conforms |
| Specific Gravity | 0.9900 - 1.0300 | 1.0040 | 0.9990 | Conforms |
| Viscosity¹ | ≥ 600 | 1989 | 1440 | Conforms |
| CHG² | 4.00-4.40 | 2.09 | 2.03 | Conforms |
| IPA² | 4.00-4.80 | 4.52 | 4.59 | Conforms |
| p-Chloroaniline³ | ≤ 50 | < 13.5 | < 13.5 | Conforms |

| | | | | |
|---|---|---|---|---|
| ¹ Units: CPS@ 25±1°C (Utilize Spindle 1 [S61] @ 60 RPM) ² Units: % w/w ³ Units: PPM | | | | |

In order to demonstrate the compatibility of DLM with chlorhexidine digluconate, a pilot batch of 4% chlorhexidine digluconate liquid solutions (with or without 4% by weight Cocamide DEA) were prepared and tested according to currently approved finished product specification criteria. The test results of the 4% CHG liquid solutions are presented in Table 6. Compared to the above solutions, CHG liquid solutions may contain, among other changes, 0.6% by weight hydroxyethylcellulose. CHG liquid solutions are more viscous than corresponding solutions above.

**Table 6. Test Results of 4% CHG Liquid Solutions.**

| **Test** | **Specifications** | **Results** | | **Conformation to Specifications** |
|---|---|---|---|---|
| | | **Cocamide DEA** | **Dimethyl Lauramide / Myristamide** | |
| Physical Appearance | Clear to very slight haze, amber liquid with no particulate | Conforms | Conforms | Conforms |
| pH | 6.00-7.50 | 6.55 | 6.26 | Conforms |
| Specific Gravity | 0.9900 - 1.0300 | 1.0117 | 1.0032 | Conforms |
| Viscosity¹ | ≥ 600 | 1798 | 1160 | Conforms |
| CHG² | 4.00-4.40 | 4.14 | 4.05 | Conforms |
| IPA² | 4.00-4.80 | 4.59 | 4.68 | Conforms |
| p-Chloroaniline³ | ≤ 50 | < 13.5 | < 13.5 | Conforms |

| | | | | |
|---|---|---|---|---|
| ¹ Units: CPS@ 25±1°C (Utilize Spindle 1 [S61] @ 60 RPM) ² Units: % w/w ³ Units: PPM | | | | |

The test results indicate that, in foaming applications, the replacement solutions containing DLM can produce greater amounts of foam than the original solutions containing cocamide DEA. The foam generated in the solutions containing DLM is more stable than that generated in the solutions containing cocamide DEA. The foam generated in DLM solutions is more evenly distributed than that in cocamide DEA solutions allowing for consistent layering of the replacement solutions and greater product efficiency. Due to DLM's higher foaming efficiency, a smaller amount of skin aseptic solution is needed to achieve the same efficacy. The replacement of cocamide DEA with DLM provides a more stable product, e.g. more stable foam in foaming applications, and allows a lower concentration of active ingredient in the final product solutions. Overall, a skin aseptic solution containing DLM is a better and much safer product than the current commercial products containing cocamide DEA.

The embodiments disclosed herein are not intended to be exhaustive or to be limiting. A skilled artisan would acknowledge that other embodiments or modifications to instant embodiments can be made without departing from the spirit or scope of the invention. The aspects of the present disclosure, as generally described herein and illustrated in the figures, can be arranged, substituted, combined, separated, and designed in a wide variety of different configurations, all of which are contemplated herein.

The use of the terms "a," "an," "the," and similar referents in the context of describing the present invention (especially in the context of the claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. Use of the term "about" is intended to describe values either above or below the stated value in a range of approximately ±10%; in other embodiments, the values may range in value above or below the stated value in a range of approximately ±5%; in other embodiments, the values may range in value above or below the stated value in a range of approximately ±2%; in other embodiments, the values may range in value above or below the stated value in a range of approximately ±1%. The preceding ranges are intended to be made clear by context, and no further limitation is implied. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise stated. No language in the specification should be construed as indicating any non-claimed element as essential to the practice of the invention.

All references cited herein are incorporated by reference in their entireties. The present invention may be embodied in other specific forms without departing from the spirit or essential attributes thereof, and, accordingly, reference should be made to the appended claims, rather than to the foregoing specification, as indicating the scope of the invention.

### I disclose:

1. A foaming disinfecting composition, comprising:
   (a) up to about 21% by weight of an antiseptic agent;
   (b) up to about 10% by weight of dimethyl lauramide/myristamide;
   (c) from about 5% to about 95% by weight of water;
   (d) optionally from about 0.00005% to about 5% by weight of a fragrance; and
   (e) optionally from about 0.00005% to about 1% by weight of a colorant.
2. The composition of statement 1, wherein the antiseptic agent includes chlorhexidine digluconate.
3. The composition of statement 2, wherein chlorhexidine digluconate is present in an amount from about 0.5% by weight to about 5% by weight, and wherein dimethyl lauramide/myristamide is present in any amount by weight.
4. The composition of statement 3, wherein a foam is produced having a density of about 0.1 g/cc to about 1.5 g/cc and/or average bubble number distribution of about 1.0x10⁵/cc to about 1.0x10⁵/cc producing stable foaming.
5. The composition of statement 4, wherein a foam is produced having a density of about 0.25±0.05 g/cc and/or average bubble number distribution of 5.0x10⁶±1.0x10⁶/cc producing stable foaming over at least 15 minutes.
6. The composition of statement 2, wherein chlorhexidine digluconate is present in an amount from about 0.75% by weight to about 0.85% by weight, and wherein dimethyl lauramide/myristamide is present in any amount by weight.
7. A foaming disinfecting composition, comprising:
   (a) up to about 21% by weight of an antiseptic agent;
   (b) up to about 10% by weight of dimethyl lauramide/myristamide;
   (c) from about 5% to about 95% by weight of water;
   (d) optionally from about 0.05% to about 5% by weight of a fragrance; and
   (e) optionally from about 0.05% to about 1% by weight of a colorant;
   wherein the composition is essentially free of carcinogen.
8. The composition of statement 7, wherein the antiseptic agent is chlorhexidine digluconate.
9. The composition of statement 8, wherein chlorhexidine digluconate is present in an amount from about 0.5% by weight to about 5% by weight, and wherein dimethyl lauramide/myristamide is present in any amount by weight.
10. The composition of statement 9, wherein a foam is produced having a density of about 0.1 g/cc to about 1.5 g/cc and/or average bubble number distribution of about 1.0x10⁵/cc to about 1.0x10⁵/cc producing stable foaming.
11. The composition of statement 10, wherein a foam is produced having a density of about 0.25±0.05 g/cc and/or average bubble number distribution of 5.0x10⁶±1.0x10⁶/cc producing stable foaming over at least 15 minutes.
12. The disinfecting composition of statement 9, wherein the carcinogen includes cocamide-DEA.
13. The disinfecting composition of statement 7, wherein the carcinogen includes cocamide-DEA.
14. The disinfecting composition of statement 7, wherein the carcinogen includes diethanolamine.
15. The disinfecting composition of statement 7, wherein the carcinogen includes ethylene-oxide.
16. A method of providing substantial antimicrobial effectiveness on skin, comprising administering an effective amount of the disinfecting composition of statement 1 to human skin.
17. A method of providing substantial antimicrobial effectiveness on skin, comprising administering an effective amount of the disinfecting composition of statement 7 to human skin.

## Claims

1. A foaming disinfecting composition, comprising:
(a) up to about 21% by weight of an antiseptic agent;
(b) up to about 10% by weight of dimethyl lauramide/myristamide;
(c) from about 5% to about 95% by weight of water;
(d) optionally from about 0.00005% to about 5% by weight of a fragrance; and
(e) optionally from about 0.00005% to about 1% by weight of a colorant.

2. The composition of claim 1, wherein the antiseptic agent includes chlorhexidine digluconate.

3. The composition of claim 2, wherein chlorhexidine digluconate is present in an amount from about 0.5% by weight to about 5% by weight, and wherein dimethyl lauramide/myristamide is present in any amount by weight.

4. The composition of any one of claims 1 - 3, wherein a foam is produced having a density of about 0.1 g/cc to about 1.5 g/cc and/or average bubble number distribution of about 1.0x10⁵/cc to about 1.0x10⁵/cc producing stable foaming.

5. The composition of claim 4, wherein a foam is produced having a density of about 0.25±0.05 g/cc and/or average bubble number distribution of 5.0x10⁶±1.0x10⁶/cc producing stable foaming over at least 15 minutes.

6. The composition of claim 2, wherein chlorhexidine digluconate is present in an amount from about 0.75% by weight to about 0.85% by weight, and wherein dimethyl lauramide/myristamide is present in any amount by weight.

7. The foaming disinfecting composition of claim 1, comprising:
(a) up to about 21% by weight of an antiseptic agent;
(b) up to about 10% by weight of dimethyl lauramide/myristamide;
(c) from about 5% to about 95% by weight of water;
(d) optionally from about 0.05% to about 5% by weight of a fragrance; and
(e) optionally from about 0.05% to about 1% by weight of a colorant;
wherein the composition is essentially free of carcinogen.

8. The composition of claim 7, wherein the antiseptic agent is chlorhexidine digluconate.

9. The composition of claim 8, wherein chlorhexidine digluconate is present in an amount from about 0.5% by weight to about 5% by weight, and wherein dimethyl lauramide/myristamide is present in any amount by weight.

10. The composition of any one of claims 7 - 9, wherein a foam is produced having a density of about 0.1 g/cc to about 1.5 g/cc and/or average bubble number distribution of about 1.0x10⁵/cc to about 1.0x10⁵/cc producing stable foaming.

11. The composition of claim 10, wherein a foam is produced having a density of about 0.25±0.05 g/cc and/or average bubble number distribution of 5.0x10⁶±1.0x10⁶/cc producing stable foaming over at least 15 minutes.

12. The disinfecting composition of any one of claims 7 - 11, wherein the carcinogen includes cocamide-DEA.

13. The disinfecting composition of any one of claims 7 - 12, wherein the carcinogen includes diethanolamine.

14. The disinfecting composition of any one of claims 7 - 13, wherein the carcinogen includes ethylene-oxide.

15. The disinfecting composition of any one of claims 1 to 14 for use in a method of providing substantial antimicrobial effectiveness on skin, the use comprising administering an effective amount of the disinfecting composition of any one of claims 1 to 14 to human skin.
